# EUROPÄISCHE PATENTSCHRIFT

(11) **EP 0 455 626 B1**
(45) Veröffentlichungstag und Bekanntmachung des Hinweises auf die Patenterteilung: **08.03.1995**
(21) Anmeldenummer: 91890091.1
(22) Anmeldetag: 29.04.1991
(51) Int. Cl.: A61B 10/00, A61M 5/158

(54) **Biopsieeinrichtung**
Biopsy device
Dispositif de biopsie

(30) Priorität: 03.05.1990 AT 1005/90
(43) Veröffentlichungstag der Anmeldung: 06.11.1991
(73) Patentinhaber: IMMUNO Aktiengesellschaft, A-1221 Wien (AT)
(72) Erfinder: Dinges, Hans Peter, Dr., A-8045 Graz (AT); Zatloukal, Kurt, Dr., A-8010 Graz (AT); Habison, Georg, Dr., A-1040 Wien (AT)
(74) Vertreter: Wolfram, Gustav, Dipl.-Ing.

(56) Entgegenhaltungen:
- EP-A- 0 221 007
- EP-A- 0 243 341
- DE-A- 3 522 782
- DE-U- 8 623 592
- FR-A- 2 378 528
- US-A- 2 426 535
- US-A- 3 477 423
- US-A- 4 224 943
- US-A- 4 693 257

## Beschreibung

Die Erfindung betrifft eine Biopsieeinrichtung zur Gewinnung einer Gewebeprobe und Applikation mindestens einer Substanz in einem Arbeitsgang, mit einer einen Biopsiekanal bildenden und eine Gewebe abtrennende Schneide aufweisenden Biopsiekanüle und mit einem Applikationskanal zur Applikation einer Blutgerinnungssubstanz.

Eine Biopsieeinrichtung dieser Art gemäß dem Oberbegriff des Anspruchs 1 ist aus der EP-A 0 243 341 bekannt.

Diese bekannte Biopsieeinrichtung zur Saugbiopsie ist gemäß einer speziellen Ausführungsform von zwei ineinander geschobenen Rohren gebildet, wobei das Außenrohr einen Kreisquerschnitt aufweist und das Innenrohr vom Außenrohr über einen Teilumfang seines Querschnitts über seine ganze Länge beabstandet ist. Das Innenrohr dient zur Aufnahme der Gewebeprobe mit Hilfe einer Saugeinrichtung und der zwischen dem Innenrohr und dem Außenrohr gebildete Raum dient zur Applikation einer Substanz, vorzugsweise einer Blutgerinnungssubstanz zur Versiegelung des von der Biopsieeinrichtung im Gewebe gebildeten Defektes.

Die Erfindung bezweckt eine Weiterentwicklung dieser bekannten Biopsieeinrichtung dahingehend, daß die Herstellung der Biopsieeinrichtung wesentlich vereinfacht ist; insbesondere sollen herkömmliche Biopsienadeln unterschiedlicher Bauart in einfacher Weise mit einem Applikationskanal ausgestattet werden können, ohne daß bauliche Veränderungen und spezielle Ausbildungen an herkömmlichen Biopsienadeln erforderlich sind. So soll es mit der erfindungsgemäßen Biopsieeinrichtung möglich sein, nicht nur Biopsienadeln für die Saugbiopsie (mit oder ohne Stilett) mit einem Applikationskanal auszustatten,sondern auch Schneidbiopsienadeln (mit oder ohne Stilett) damit zu versehen, beispielsweise Schneidbiopsienadeln gemäß US-A 3 477 423.

Diese Aufgabe wird erfindungsgemäß mit den im kennzeichnenden Teil des Anspruchs 1 angegebenen Merkmalen gelöst.

Diese erfindungsgemäße Biopsieeinrichtung gestattet die Anordnung eines Applikationsrohres an herkömmlichen Biopsienadeln unabhängig von deren Funktionsweise (Saug- oder Schneidbiopsie, mit oder ohne Stilett bzw. Systeme mit Trokar) und unabhängig von deren Länge. Dadurch, daß die den Biopsiekanal bildende Biopsiekanüle das Anschlußstück durchsetzt, läßt sich in einfacher Weise eine Dichtung zwischen dieser Biopsiekanüle und dem Anschlußstück zur Abdichtung und Fixierung des Applikationskanales anordnen, ohne daß bauliche Veränderungen an der Biopsiekanüle vorgenommen werden müssen. Der zwischen der Biopsiekanüle und dem Applikationsrohr gebildete, vorteilhaft ringförmige, Spalt kann sehr eng bemessen sein, sodaß sich ein nur geringfügig gegenüber herkömmlichen Biopsiekanülen vergrößerter Außendurchmesser der erfindungsgemäßen Biopsieeinrichtung ergibt. Trotzdem läßt sich die zu applizierende Substanz einwandfrei dem Körpergewebe zuführen. Ein besonderer Vorteil der erfindungsgemäßen Biopsieeinrichtung ist darin zu sehen, daß Biopsienadeln mit im Inneren der Biopsiekanüle angeordneter Schneideinrichtung, wie z.B.Tru-Cut Biopsienadeln, mit einem Applikationskanal versehen werden können, sodaß auch bei dieser Art von Biopsienadeln der Vorteil des Applizierens einer Substanz, insbesondere einer Blutgerinnungssubstanz, voll zum Tragen kommt.

Gemäß einer bevorzugten Ausführungsform ist das Anschlußstück T- oder Y-förmig ausgebildet und weist einen geraden Rohrteil auf, durch den die Biopsiekanüle hindurchragt, an dessen einem Ende das Applikationsrohr dichtend fixiert ist und dessen gegenüberliegendes Ende gegenüber der Biopsiekanüle abgedichtet ist und mündet in den geraden Rohrteil ein vom geraden Rohrteil auskragender Rohrteil, an den mindestens eine die Substanz(en) zum Applikationsrohr fördernde Leitung anschließbar ist.

Hiebei ist zweckmäßig zur Abdichtung des Anschlußstückes gegenüber der Biopsiekanüle an einem konzentrisch zur Biopsiekanüle liegenden Ende des Anschlußstückes eine Mutter mit einem zentralen Durchlaß für die Biopsiekanüle und eine zwischen der Mutter und dem Anschlußstück angeordnete und durch Festziehen der Mutter verformbare Dichtung vorgesehen, wobei vorteilhaft die Mutter als Überwurfmutter ausgebildet ist.

Vorteilhaft ist die verformbare Dichtung aus elastischem Kunststoff, wie Silikon oder Polyurethan gebildet.

Gemäß einer bevorzugten Ausführungsform ist das Anschlußstück mit dem Applikationsrohr durch eine Klebeverbindung verbunden oder direkt in das Anschlußstück eingegossen.

Eine weitere bevorzugte Ausführungsform ist dadurch gekennzeichnet, daß das Anschlußstück mit dem Applikationsrohr durch eine mechanische Verbindung, wie eine Schraub-, Steck- oder arretierbare Steckverbindung verbunden ist.

Dies bietet den Vorteil, daß der Durchmesser und die Länge des Applikationsrohres für ein- und dasselbe Anschlußstück frei wählbar sind und entsprechend der mit einem Applikationsrohr zu versehenden Biopsienadel gewählt werden können.

Vorteilhaft ist der auskragende Rohrteil des Anschlußstückes mit einem Luerkonus versehen.

Wie bereits eingangs erwähnt, ergeben sich besondere Vorteile, wenn die Biopsiekanüle und das Applikationsrohr Kreisquerschnitt aufweisen, wobei zweckmäßig der Innendurchmesser des Applikationsrohres zum Außendurchmesser der Biopsiekanüle in einem Verhältnis von 1,05 bis 1,5 steht.

Die erfindungsgemäße Biopsieeinrichtung ist zur Saugbiopsie geeignet, wenn die Biopsiekanüle an eine Saugeinrichtung anschließbar ist.

Für die Zwecke der Schneidbiopsie ist vorteilhaft innerhalb der Biopsiekanüle eine mit einer schneidenden oder das Gewebe durchdringenden Spitze versehene Nadel längsverschieblich gelagert, die an ihrem vorderen Ende mit einer Ausnehmung zur Aufnahme einer Gewebeprobe versehen ist, wobei die Ausnehmung von einer Lage innerhalb der Biopsiekanüle in eine außerhalb der Biopsiekanüle liegende Position und retour verschiebbar ist.

Ein besonders leichtes Eindringen der Biopsieeinrichtung wird ermöglicht, wenn das vordere Ende des Applikationsrohres zur Längsachse geneigt ausgebildet ist.

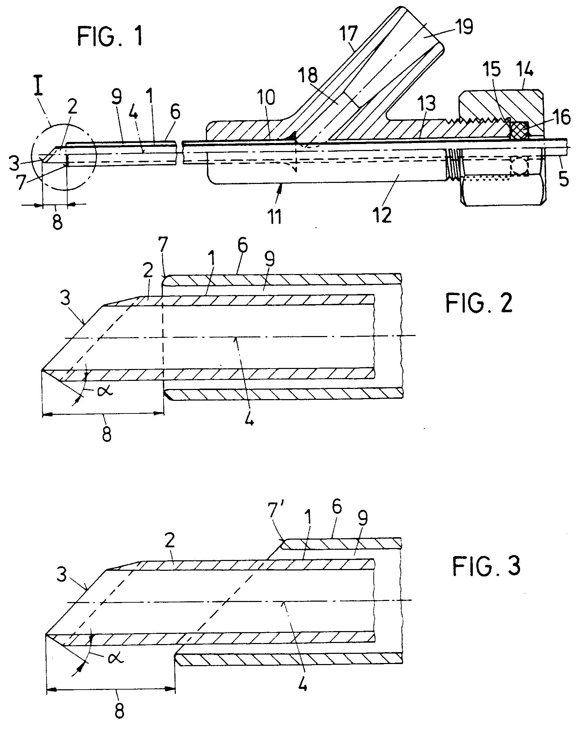

Die Erfindung ist nachfolgend anhand eines in der Zeichnung dargestellten Ausführungsbeispiels näher erläutert, wobei Fig. 1 einen Längsschnitt durch die vergrößert dargestellte Biopsieeinrichtung und Fig. 2 ein Detail I der Fig. 1 im vergrößerten Maßstab zeigen. Fig. 3 zeigt in zu Fig. 2 analoger Darstellung eine geänderte Ausführungsform.

Die Biopsieeinrichtung weist eine Biopsiekanüle 1 mit vorteilhaft kreisförmigem Querschnitt auf. Das vordere Ende 2 dieser Biopsiekanüle 1 ist als Schneide 3 ausgebildet, die zur Längsachse 4 der Biopsiekanüle 1 entweder rechtwinkelig oder schräg bzw. gekrümmt angeordnet ist.

Das hintere Ende 5 der Biopsiekanüle 1 ist an sich in bekannter Weise an eine Saugeinrichtung anschließbar, was nicht näher dargestellt ist.

Dieses hintere Ende 5 könnte auch mit einem Kontaktmechanismus zur Begrenzung der Punktionstiefe, ähnlich wie er in der EP-A 243 341 dargestellt ist, ausgestattet sein.

Über die Biopsiekanüle 1 ist ein ebenfalls einen zweckmäßig kreisförmigen Querschnitt aufweisendes Applikationsrohr 6 geschoben, dessen Länge kürzer ist als die der Biopsiekanüle 1, d.h. das vordere Ende 7 des Applikationsrohres 6 endet im geringen Abstand 8 hinter der Schneide 3 der Biopsiekanüle 1. Dieses Ende 7 ist (entsprechend Ausführungsbeispiel Fig. 2) rechtwinkelig zur Längsachse 4 ausgebildet und schräg angeschliffen, wobei das vorderste Ende abgerundet ausgebildet ist, sodaß das Gewebe von dem Applikationsrohr 6 nach außen gedrängt wird und nicht in den zwischen Biopsiekanüle 1 und Applikationsrohr 6 gebildeten ringförmigen Applikationskanal 9 eindringen kann.

Das hintere leicht aufgebördelte Ende 10 des Applikationsrohres 6 ist in ein Anschlußstück 11 eingegossen - es könnte auch eingeklebt sein -, welches Anschlußstück 11 einen geradlinigen, sich in Richtung der Längsachse 4 erstreckenden Rohrteil 12 mit einem zentralen Durchgangskanal 13 für die Biopsiekanüle 1 aufweist. Am hinteren Ende des Rohrteiles 12 ist eine Überwurfmutter 14 aufgeschraubt. Zwischen der Überwurfmutter 14 und dem hinteren Stirnende 15 dieses Rohrteiles 12 ist eine elastisch verformbare Dichtung 16 aus Kunststoff, vorzugsweise aus Silikon oder Polyurethan, angeordnet. Ein Festziehen der Überwurfmutter gegenüber dem Rohrteil 12 bewirkt ein Verformen der elastischen Dichtung 16 und damit ein dichtes Anliegen und eine Fixierung an der Außenseite der Biopsiekanüle 1.

An dem geraden, mit dem zentralen Durchgangskanal 13 versehenen Rohrteil 12 des Anschlußstückes 11 ist ein seitlich auskragender Rohrteil 17 angeformt, dessen Innenkanal 18 in den zentralen Durchgangskanal 13 mündet. Am äußeren Ende ist dieser Rohrteil 17 zweckmäßig mit einem Luerkonus 19 zum Ansetzen einer Applikationseinrichtung versehen. Die Applikationseinrichtung kann auch mit einer Schraub-, Steck- oder arretierbaren Steckverbindung am Rohrteil 17 befestigbar sein.

Gemäß der in Fig. 3 dargestellten Ausführungsform ist das vordere Ende 7' des Applikationsrohres 6 abgeschrägt ausgebildet. Es erstreckt sich etwa parallel zur Schneide 3 der Biopsiekanüle 1. Wie aus den Fig. 2 und 3 ersichtlich, ist der Anschliff des Endes 2 der Biopsiekanüle 1 unter einem Winkel alpha getroffen, der spitzer ist als der Winkel zwischen der Endfläche des Applikationsrohres 6 und dessen Längsachse 4.

Die Erfindung beschränkt sich nicht auf die in der Zeichnung dargestellten Ausführungsbeispiele, sondern kann modifiziert werden. Beispielsweise ist es möglich, anstelle der dargestellten Verbindung zwischen Applikationsrohr 6 und Anschlußstück 11 eine mechanische Verbindung, wie eine Schraub-, Steck- oder arretierbare Steckverbindung vorzusehen, sodaß in ein- und dasselbe Anschlußstück 11 unterschiedliche Applikationsrohre 6 einsetzbar sind, deren Innendurchmesser optimal an den Außendurchmesser der Biopsiekanüle bzw. deren Länge an die Länge der Biopsiekanüle angepaßt ist. Anstelle der in der Zeichnung dargestellten Saug-Biopsiekanüle 1 kann auch eine Schneid-Biopsiekanüle vorgesehen sein, wie sie z.B. in der US-A 3 477 423 gezeigt ist.

## Patentansprüche

1. Biopsieeinrichtung zur Gewinnung einer Gewebeprobe und Applikation mindestens einer Substanz in einem Arbeitsgang, mit einer einen Biopsiekanal bildenden und eine Gewebe abtrennende Schneide (3) aufweisenden Biopsiekanüle (1) und mit einem Applikationskanal (9) zur Applikation einer Blutgerinnungssubstanz, der von einem über die Biopsiekanüle (1) geschobenen Applikationsrohr (6) begrenzt ist, dessen vorderes Ende (7) gegenüber der Schneide (3) der Biopsiekanüle (1) zurückversetzt ist, und an dessen gegenüberliegendem Ende ein damit dicht verbundenes Anschlußstück (11) zum Anschluß mindestens einer die Substanz(en) zum Applikationskanal (9) fördernden Leitung vorgesehen ist, dadurch gekennzeichnet, daß die Biopsiekanüle (1) das Anschlußstück (11) unter Vorsehen einer Dichtung (16) durchsetzt, und daß der Applikationskanal (9) als die einen Kreisquerschnitt aufweisende Biopsiekanüle (1) umgebender, im Querschnitt ringförmiger Spalt ausgebildet ist.

2. Biopsieeinrichtung nach Anspruch 1, dadurch gekennzeichnet, daß das Anschlußstück (11) T- oder Y-förmig ausgebildet ist und einen geraden Rohrteil (12) aufweist, durch den sich die Biopsiekanüle (1) hindurcherstreckt, an dessen einem Ende das Applikationsrohr (6) dichtend fixiert ist, und an dessen gegenüberliegendem Ende die Dichtung (16) gegenüber der Biopsiekanüle (1) vorgesehen ist, und daß in den geraden Rohrteil (12) ein vom geraden Rohrteil auskragender Rohrteil (17) mündet, an den mindestens eine die Substanz(en) zum Applikationsrohr (6) fördernde Leitung anschließbar ist.

3. Biopsieeinrichtung nach Anspruch 1 oder 2, dadurch gekennzeichnet, daß zur Abdichtung des Abschlußstückes (11) gegenüber der Biopsiekanüle (1) an einem konzentrisch zur Biopsiekanüle (1) liegenden Ende (15) des Anschlußstückes (11) eine Mutter (14) mit einem zentralen Durchlaß für die Biopsiekanüle (1) und eine zwischen der Mutter (14) und dem Anschlußstück (11) angeordnete und durch Festziehen der Mutter (14) verformbare Dichtung (16) vorgesehen ist.

4. Biopsieeinrichtung nach Anspruch 3, dadurch gekennzeichnet, daß die Mutter als Überwurfmutter (14) ausgebildet ist.

5. Biopsieeinrichtung nach Anspruch 3 oder 4, dadurch gekennzeichnet, daß die verformbare Dichtung (16) aus elastischem Kunststoff, wie Silikon oder Polyurethan, gebildet ist.

6. Biopsieeinrichtung nach einem oder mehreren der Ansprüche 1 bis 5, dadurch gekennzeichnet, daß das Anschlußstück (11) mit dem Applikationsrohr (6) durch eine Klebeverbindung verbunden oder direkt in das Anschlußstück eingegossen ist.

7. Biopsieeinrichtung nach einem oder mehreren der Ansprüche 1 bis 5, dadurch gekennzeichnet, daß das Anschlußstück (11) mit dem Applikationsrohr (6) durch eine mechanische Verbindung, wie eine Schraub-, Steck- oder arretierbare Steckverbindung verbunden ist.

8. Biopsieeinrichtung nach einem oder mehreren der Ansprüche 2 bis 7, dadurch gekennzeichnet, daß der auskragende Rohrteil (17) des Anschlußstückes (11) mit einem Luerkonus (19) versehen ist.

9. Biopsieeinrichtung nach einem der mehreren der Ansprüche 1 bis 8, dadurch gekennzeichnet, daß das Applikationsrohr (6) Kreisquerschnitt aufweist.

10. Biopsieeinrichtung nach Anspruch 9, dadurch gekennzeichnet, daß der Innendurchmesser des Applikationsrohres (6) zum Außendurchmesser der Biopsiekanüle (1) in einem Verhältnis von 1,05 bis 1,5 steht.

11. Biopsieeinrichtung nach einem oder mehreren der Ansprüche 1 bis 10, dadurch gekennzeichnet, daß die Biopsiekanüle (1) an eine Saugeinrichtung anschließbar ist.

12. Biopsieeinrichtung nach einem oder mehreren der Ansprüche 1 bis 10, dadurch gekennzeichnet, daß innerhalb der Biopsiekanüle (1) eine mit einer schneidenden oder das Gewebe durchdringenden Spitze versehene Nadel längsverschieblich gelagert ist, die an ihrem vorderen Ende mit einer Ausnehmung zur Aufnahme einer Gewebeprobe versehen ist, wobei die Ausnehmung von einer Lage innerhalb der Biopsiekanüle in eine außerhalb der Biopsiekanüle liegende Position und retour verschiebbar ist.

13. Biopsieeinrichtung nach einem oder mehreren der Ansprüche 1 bis 12, dadurch gekennzeichnet, daß das vordere Ende (7') des Applikationsrohres (6) zur Längsachse (4) geneigt ausgebildet ist.

## Claims

1. A biopsy device for obtaining a tissue sample and for applying at least one substance in one operation, said biopsy device comprising a biopsy cannula (1) forming a biopsy channel and having a cutting edge (3) for cutting off tissue, and an application channel (9) for applying a blood-clotting substance, which application channel is delimited by an application tube (6) slipped over the biopsy cannula (1), the front end (7) of the application tube being rearwardly offset relative to the cutting edge (3) of the biopsy cannula (1), whereas at its opposite end there is provided a tightly joined connecting piece (11) for connection with at least one duct conveying the substance(s) to the application channel (9), characterised in that the biopsy cannula (1) traverses the connecting piece (11) under provision of a sealing (16), and in that the application channel (9) is designed as a gap of annular cross-section surrounding the biopsy cannula (1) which has a circular cross-section.

2. A biopsy device according to claim 1, characterised in that the connecting piece (11) is T or Y shaped and comprises a straight tube portion (12) through which the biopsy cannula (1) extends, the application tube (6) being sealingly fixed to one end of the straight tube portion, whereas at its opposite end the sealing (16) relative to the biopsy cannula (1) is provided, and in that a tube portion (17) cantilevering from the straight tube portion enters into in the straight tube portion (12), to which cantilevering tube portion at least one duct conveying the substance(s) to the application tube (6) is connectable.

3. A biopsy device according to claim 1 or 2, characterised in that a nut (14) having a central throughhole for the biopsy cannula (1), and a seal (16) arranged between the nut (14) and the connecting piece (11) and deformable by tightening of the nut (14) are provided on an end (15) of the connecting piece (11) located concentrical to the biopsy cannula (1), for sealing the connecting piece (11) relative to the biopsy cannula (1).

4. A biopsy device according to claim 3, characterised in that the nut is designed as a box nut (14).

5. A biopsy device according to claim 3 or 4, characterised in that the deformable seal (16) is formed of elastic synthetic material, such as silicone or polyurethane.

6. A biopsy device according to one or several of claims 1 to 5, characterised in that the connecting piece (11) is connected with the application tube (6) by a glue connection or is directly cast into the connecting piece.

7. A biopsy device according to one or several of claims 1 to 5, characterised in that the connection piece (11) is connected with the application tube (6) by a mechanical connection, such as a screw, plug or lockable plug connection.

8. A biopsy device according to one or several of claims 2 to 7, characterised in that the cantilevering tube portion (17) of the connection piece (11) is provided with a Luer cone (19).

9. A biopsy device according to one or several of claims 1 to 8, characterised in that the application tube (6) has a circular cross-section.

10. A biopsy device according to claim 9, characterised in that the ratio of the inner diameter of the application tube (6) relative to the outer diameter of the biopsy cannula (1) ranges from 1.05 to 1.5.

11. A biopsy device according to one or several of claims 1 to 10, characterised in that the biopsy cannula (1) is connectable to a suction device.

12. A biopsy device according to one or several of claims 1 to 10, characterised in that a needle having a cutting tip or a tissue-penetrating tip is longitudinally displaceably mounted within the biopsy cannula (1), which needle is provided with a recess at its front end so as to accommodate a tissue sample, the recess being displaceable from a position located within the biopsy cannula to a position located outside of the biopsy cannula and back.

13. A biopsy device according to one or several of claims 1 to 12, characterised in that the front end (7') of the application tube (6) is designed so as to be inclined relative to the longitudinal axis (4).

## Revendications

1. Dispositif de biopsie destiné au prélèvement d'une pièce tissulaire et à l'administration d'au moins une substance en une seule opération, comportant une canule de biopsie (1) formant un canal de biopsie et présentant un tranchant (3) d'ablation tissulaire ainsi qu'un canal d'administration (9) destiné à l'administration d'une substance coagulante et qui est limité par un tube d'administration (6) enfilé sur la canule de biopsie (1) et dont l'extrémité avant (7) est en retrait par rapport au tranchant (3) de la canule de biopsie (1) et à l'extrémité opposée duquel il est prévu un raccord (11) relié de façon étanche à ce dernier pour permettre le raccord d'au moins un conduit transportant la (les) substance(s) au canal d'administration (9), caractérisé en ce que la canule de biopsie (1) traverse le raccord (11) cependant qu'est prévu un joint (16) et en ce que le canal d'administration (9) est conçu sous forme de fente de section transversale annulaire, entourant la canule de biopsie (1) présentant une section transversale circulaire.

2. Dispositif de biopsie selon la revendication 1, caractérisé en ce que le raccord (11) est en T ou en Y et présente une partie tubulaire droite (12) à travers laquelle s'étend la canule de biopsie (11), à une extrémité duquel le tube d'administration (6) est fixé de façon étanche et à l'extrémité opposée duquel le joint (16) est prévu par rapport à la canule de biopsie (1) et en ce qu'une partie tubulaire (17) en saillie par rapport à la partie tubulaire droite et à laquelle peut être raccordé au moins un conduit transportant la (les) substance(s) au tube d'administration (6) débouche dans la partie tubulaire droite (12).

3. Dispositif de biopsie selon la revendication 1 ou 2, caractérisé en ce qu'il est prévu, pour l'étanchéification du raccord (11) par rapport à la canule de biopsie (1), à une extrémité (15) du raccord (11) concentrique par rapport à la canule de biopsie (1), un écrou (14) à trou central pour la canule de biopsie (1) et un joint (16) disposé entre l'écrou (14) et le raccord (11) et déformable par serrage de l'écrou (14).

4. Dispositif de biopsie selon la revendication 3, caractérisé en ce que l'écrou a la forme d'un écrou-raccord (14).

5. Dispositif de biopsie selon la revendication 3 ou 4, caractérisé en ce que le joint déformable (16) est en une matière plastique élastique, telle que silicone ou polyuréthanne.

6. Dispositif de biopsie selon une ou plusieurs des revendications 1 à 5, caractérisé en ce que le raccord (11) est collé au tube d'administration (6) ou que ce dernier est coulé directement dans le raccord.

7. Dispositif de biopsie selon une ou plusieurs des revendications 1 à 5, caractérisé en ce que le raccord (11) est fixé mécaniquement au tube d'administration (6), par exemple par vis, connecteur ou connecteur blocable.

8. Dispositif de biopsie selon une ou plusieurs des revendications 2 à 7, caractérisé en ce que la partie tubulaire en saillie (17) du raccord (11) est dotée d'un cône de Luer (19).

9. Dispositif de biopsie selon une ou plusieurs des revendications 1 à 8, caractérisé en ce que le tube d'administration (6) présente une section transversale circulaire.

10. Dispositif de biopsie selon la revendication 9, caractérisé en ce que le diamètre interne du tube d'administration (6) est dans un rapport de 1,05 à 1,5 au diamètre externe de la canule de biopsie (1).

11. Dispositif de biopsie selon une ou plusieurs des revendications 1 à 10, caractérisé en ce que la canule de biopsie (1) peut être connectée à un dispositif d'aspiration.

12. Dispositif de biopsie selon une ou plusieurs des revendications 1 à 10, caractérisé en ce qu'une aiguille dotée d'une pointe tranchante ou pénétrant dans le tissu et présentant à son extrémité avant un évidement destiné à recueillir une pièce tissulaire est disposée de façon à coulisser longitudinalement à l'intérieur de la canule de biopsie (1), l'évidement étant coulissable d'une position à l'intérieur de la canule de biopsie à une position à l'extérieur de la canule de biopsie et vice versa.

13. Dispositif de biopsie selon une ou plusieurs des revendications 1 à 12, caractérisé en ce que l'extrémité avant (7') du tube d'administration (6) est oblique par rapport à l'axe longitudinal (4).
